# EUROPEAN PATENT SPECIFICATION

(11) **EP 2 416 892 B1**
(45) Date of publication and mention of the grant of the patent: **06.03.2013**
(21) Application number: 10713757.2
(22) Date of filing: 07.04.2010
(51) Int. Cl.: B05D 3/06, B29C 59/16, A61F 2/16

(54) **INTRAOCULAR LENS INJECTOR WITH HYDROPHILIC COATING**
INTRAOKULARLINSENINJEKTOR MIT HYDROPHILER BESCHICHTUNG
INJECTEUR DE LENTILLE INTRAOCULAIRE AVEC REVÊTEMENT HYDROPHILE

(30) Priority: 07.04.2009 US 167220 P; 05.04.2010 US 754122
(43) Date of publication of application: 15.02.2012
(73) Proprietor: Bausch & Lomb Incorporated, Rochester, NY 14604-2701 (US)
(72) Inventor: JANI, Dharmendra, Fairport NY 14450 (US); CHITRE, Kaustubh, S., Goleta, California 93117 (US)
(74) Representative: Vossius & Partner
(86) International application number: PCT/US2010/030158
(87) International publication number: WO 2010/118080

(56) References cited:
- US-A- 5 296 273
- US-A1- 2004 258 850
- US-A1- 2004 267 359
- US-A1- 2006 088 666

## Description

### Field of the Invention

The invention relates to an injector for implanting an intraocular lens into an eye of a patient according to claim 1, and to methods for making such injector. Portions of the injector include a hydrophilic coating component to facilitate the insertion of a foldable intraocular lens into an eye.

### Background of the Invention

IOLs are artificial lenses used to replace natural crystalline lenses of patients' when their natural lenses are diseased or otherwise impaired. IOLs may be placed in either the posterior chamber or the anterior chamber of an eye. IOLs come in a variety of configurations and materials. Various instruments and methods for implanting such IOLs in an eye are known. Typically, an incision is made in a patient's cornea and an IOL is inserted into the eye through the incision. In one technique, a surgeon uses surgical forceps to grasp the IOL and insert it through the incision into the eye. While this technique is still practiced today, more and more surgeons are using IOL injectors, which offer advantages such as permitting insertion of IOLs through smaller incisions. Relatively small incision sizes (e.g., less than about 3mm) are preferred over relatively large incisions (e.g., about 3.2 to 5+mm) since smaller incisions have been attributed with reduced post-surgical healing time and reduced complications such as induced astigmatism.

To fit through a small incision an IOL is typically folded and compressed within a loading or folding chamber of the IOL injector. Following release of the folded IOL into the lens capsule, the IOL unfolds and assume its original uncompressed shape. The surgeon may make minor positional adjustments to the IOL as needed. It is desirable that an IOL be released from the tip of the IOL injector in an undamaged condition and in a predictable orientation. Should an IOL be damaged or released from the injector in an incorrect orientation, a surgeon may need to remove the IOL.

A problem arises, however, as the surgeon demands even smaller incision sizes and/or the design complexities of the IOL inhibits an efficient folding of the lens. For example, the material from which the injector tube or tip is made, for example, polypropylene and the like polymeric materials, may not be compatible or suitable for passing a tightly folded IOL through an injector opening of sufficient size to fit within the incision. For example, an injector portion, particularly an injector tip, may be made of polymeric materials that have insufficient lubricity to facilitate the passage of a folded IOL through the injector portion. Often attempts to force an IOL through an injector with insufficient lubricity will lead to damage of the IOL during delivery.

One approach to enhancing the lubricity of the injector is to coat portions of the injector, particularly the injector tip with viscoelastic agents such as hyaluronic acid or HPMC. Another approach is to attach a coating to an interior surface of the injector tip to facilitate the passing of the IOL through the tip. U.S. Patent No. 5,716,364 describes adding a lubricity enhancing component to an interior surface of the injector tip. The lubricity enhancing component is said to be physically secured to the injector tip by exposing the uncoated or previously coated injector tip to an effective plasma. The plasma-exposed interior surface is said to have an enhanced ability to physically secure or bond the lubricity enhancing component relative to a substantially identical interior surface that is not plasma-exposed. In particular, the '364 Patent describes and provides examples of IOL injectors that have been subjected to both a thermal process of "blooming", i.e., a known process in which a hydrophilic or lubricant additive in a molded hydrophobic polymer migrates to the surface, and a plasma surface treatment. The plasma-exposed surface is said to have "an enhanced ability to physically secure or bond" the lubricant additive at the surface relative to a substantially similar process but without the plasma-treatment.

US 2004/0267359 discloses an apparatus for inserting intraocular lenses (IOLs) which includes a hollow tube having an interior wall defining a hollow space through which an intraocular lens may be passed from the open space into an eye. A lubricity enhancing component is covalently bonded to the hollow tube at the interior wall in an amount effective to facilitate the passage of the intraocular lens through the hollow space.

It would be advantageous to provide an IOL injector with an enhanced lubricity profile such that incision sizes as small as 3.0 mm can be used during surgical procedures. Also, lubricious coatings that exhibit prolonged shelf-stability, are stable under sterilization conditions and show little or no transfer of the coating to the IOL during delivery would provide a significant technical improvement in ocular cataract surgery.

### Summary of the Invention

An injector includes one or more polymeric portions that include a hydrophilic coating component that is effective to facilitate the passage of the IOL through the injector, particularly.the passage of the IOL through the injector tip. The degree of lubricity can be conveniently controlled by the selection of the hydrophilic coating component as well as the concentration of the hydrophilic component at the surface. Also, the hydrophilic coating component is stable on a long term basis, that is, the injector has a relatively long shelf life.

The IOL injector is prepared by a process that includes irradiating at least a portion of a polymeric, IOL injector with UV light in an environment comprising oxygen to provide a positive percent change in the atomic oxygen content of the polymer material at the surface as determined by X-ray Photoelectron Spectroscopy (XPS). A portion of or the entire irradiated portion is contacted with a solution comprising a a hydrophilic copolymer selected from poly (PVP-VA), poly (PVP-AA) or poly (PVP-DMA) to provide a solution coated portion. The solution coated portion is heated at a temperature to provide portions of the IOL injector with a shelf-stable, lubricious hydrophilic coating to facilitate delivery of an IOL from the injector with minimal damage to the lens and with little or no transfer of the hydrophilic coating to a surface of the lens.

The use of the IOL injector allows successful ocular implantation of various IOL materials such as hydrophobic acrylics, hydrophilic acrylics as well as silicone-based materials. The IOL injector allows the surgeon to implant an IOL through incisions of about 3.0 mm or less, preferably about 2.6 mm or less, and even as small as about 2.2 mm or less.

### Brief Description of the Drawings

Illustrative, non-limiting embodiments of the invention will be described by way of example with reference to the accompanying drawings, in which the same reference number is used to designate the same or similar components in different figures, and in which:
FIG. 1 is a perspective view of an exemplary injector with an attachable IOL cartridge, the injector is in a disassembled state;
FIG. 2 is a perspective view of the injector of FIG. 1 in an assembled state, ready to inject an IOL into an eye;
FIG. 3A is a perspective view of the IOL disposed on an open cartridge;
FIG. 3B is a perspective view of a closed cartridge of FIG. 3A; and

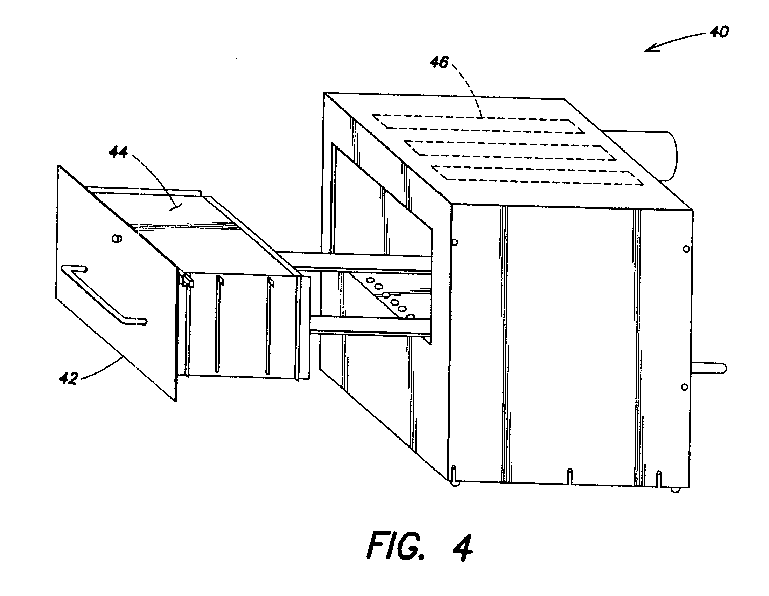

FIG. 4 is a schematic representation of the UV irradiation apparatus used to irradiate portions of an IOL injector.

### Detailed Description of the Invention

The hydrophilic coating component disposed on select portions of an IOL injector is effective to reduce the force needed to pass the IOL through the injector, particularly as the IOL is folded within the loading cartridge or as the IOL travels along the injector tip, relative to the force needed to pass an identical IOL of the same injector design, but without the hydrophilic coating component. This "reduced force" feature of the injector is particularly useful, even when no reduction in the size of the incision is obtained. The use of reduced force allows the surgeon to have more control of the rate at which the IOL is implanted into the eye, thereby reducing the risk of damage to the eye as well as the delivered lens during the surgical procedure.

The invention is directed to an IOL injector prepared by a process that includes irradiating at least a portion of a polymeric, IOL injector with UV light in an environment comprising oxygen to provide a positive percent change in the atomic oxygen content of the polymer material at the surface as determined by X-ray Photoelectron Spectroscopy (XPS). A portion of or the entire irradiated portion is contacted with a solution comprising selected from poly (PVP-VA), poly (PVP-AA) or poly (PVP-DMA) to a hydrophilic copolymer provide a solution coated portion. The solution coated portion is heated at a temperature to provide portions of the IOL injector with a shelf-stable, lubricious hydrophilic coating to facilitate delivery of an IOL from the injector with minimal damage to the lens and with little or no transfer of the hydrophilic coating to a surface of the lens.

The material from which the injector is made, or at least the portions of the injector that are coated with the hydrophilic component is made of a polymeric material, for example, a hydrophobic polymeric material, more preferably selected from polyolefins such as polypropylene and the like materials.

Although the hydrophilic coasting component preferably does not pass into the eye or to the lens during use, i.e., during the surgical procedure, hydrophilic coating components that are substantially non-irritating to ocular tissue and/or are substantially biocompatable with ocular tissue are particularly useful. The hydrophilic coating component is present in an amount effective to enhance the lubricity of an interior surface of the coated portions of the IOL injector through which the IOL travels as it is being delivered. Such hydrophilic coating components are preferably effective to provide such enhanced lubricity for relatively long periods of time, for example, for at least about 6 months or at least about 12 months. Accordingly, the IOL injector has a relatively long shelf life and can be used after being packaged, sterilized and stored for relatively long periods of time and still possess the commercial advantages of enhanced lubricity and translational stability of the hydrophilic coating, i.e.,with little or no transfer of the coating component to the surface of the IOL during storage or during delivery of the IOL.

In one embodiment, the hydrophilic coating component includes a copolymer of vinyl pyrrolidone and vinyl acetate (PVP-VA). The hydrophobic comonomer, vinyl acetate, in the PVP:VA copolymer is believed to improve upon the compatibility/wetting and hydrophobic association of the coating component with the hydrophobic surface of the substrate polymer. Preferably, the ratio of PVP:VA in the copolymer is from 30:70 to 80:20. For example, the PVP:VA copolymer can be 70:30 PVP-VA, 60:40 PVP-VA, 50:50 PVP-VA or 40:60 PVP-VA.

Another copolymer of particular interest is polyvinylpyrrolidone-polyacrylic acid (PVP-AA). Such a copolymer would have a similar ratio of PVP:AA as described for PVP:VA.

In still another embodiment, the hydrophilic coating component includes a mixture of polyvinylpyrrolidone and a copolymer of PVP-VA or PVP-AA.

Securing the hydrophilic coating component to a polymeric portion of the IOL injector is facilitated by exposing the substrate to UV light, e.g., UV-A, UV-B or UV-C, in an environment that includes oxygen, e.g., air, for a sufficient time to provide a positive percent change in the atomic oxygen content of the polymer material at the surface as determined by X-ray Photoelectron Spectroscopy (XPS). See Example Section.

An XPS surface analysis of a non-irradiated, polypropylene, IOL inserter tip will typically exhibit percent carbon values of near 100% with little or no detection of elemental oxygen. Upon UV irradiation of the tip, the percent surface carbon values decrease and the percent surface oxygen values increase, which significantly changes the chemical character of the inserter tip surface. For example, following the irradiation as described one will generally observe XPS carbon values decrease from near 100% to about 80% - 95%, and XPS oxygen values increase from near 0% to about 5% - 20%.

The increase in oxygen content is believed to modify the wettability of the IOL injector polymer, and to provide surface functionality to the IOL injector polymer through which the hydrophilic coating components is effectively secured to portions of the injector. In other words, the exposure of the IOL injector polymer provides sufficient translational stability to the hydrophilic coating component thereby inhibiting or minimizing the transfer of the coating component into the eye or onto the lens during the delivery of the lens, e.g., during surgical implantation of the lens.

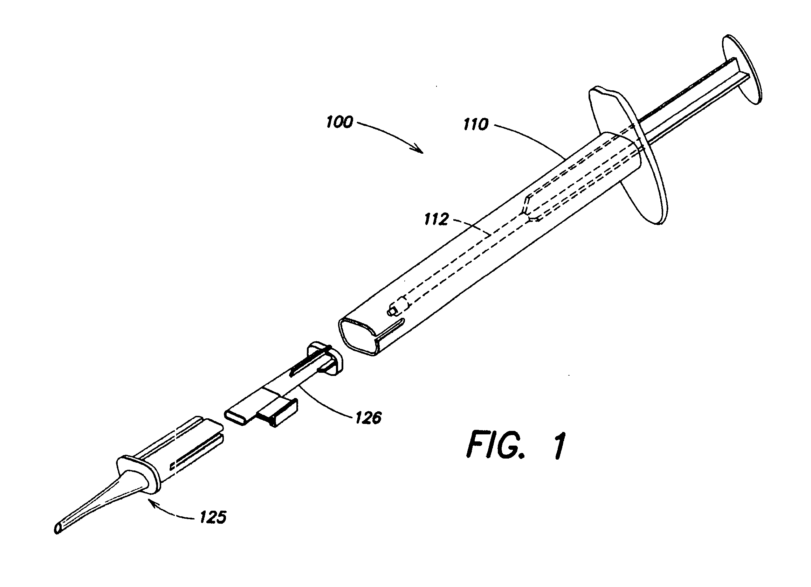

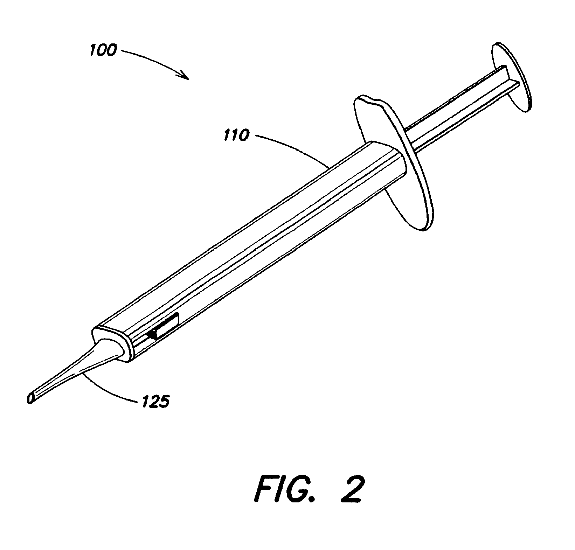

A perspective view of an IOL injector 100 is depicted in FIG. 1, as a multi-unit injector that includes a shaft 110 with an internal plunger 112, an IOL loading cartridge 126 and an injector tip 125. In one embodiment, both the loading cartridge 126 and the inserter tip 125 would have the described hydrophilic coating component disposed on an interior surface. In another embodiment, only the injector tip 126 would have the described hydrophilic coating component disposed on an interior surface. FIG.2 depicts the assembled multi-unit injector.

One of ordinary skill would recognize that the multi-unit injector could be packaged separately as in a kit ready for surgical use. In this instance, surgical personnel would assemble the components or units of the injector prior to the surgical procedure. In some embodiments, the subassembly is configured to facilitate loading of an IOL injector with an IOL by limiting the manipulation of the subassembly by surgical staff to: (1) closing of the cartridge to fold the IOL, and (2) connection of the cartridge with remaining components of an injector. Alternatively, one of ordinary skill would recognize that the multi-unit injector could be pre-assembled and packaged ready-for-use by surgical personnel. For example, the surgical personnel would only have to close the cartridge to fold the already loaded IOL, input an aqueous solution to wet the hydrophilic coating component and implement the preloaded IOL into the eye.

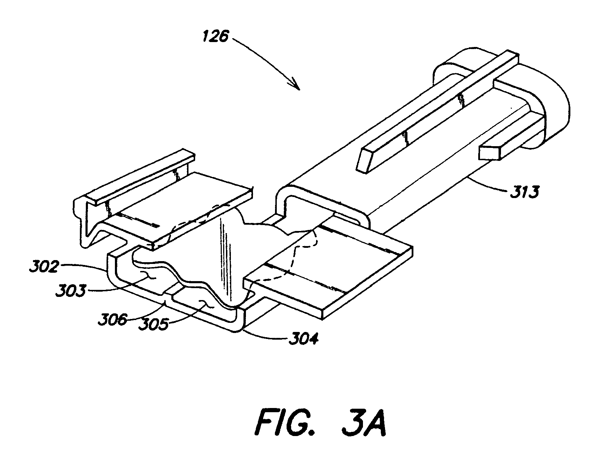

FIG. 3A is a perspective view of the IOL disposed on an open cartridge 126. The cartridge may be any suitable conventional cartridge. The lens cartridge 126 includes a first portion 302 comprising a first lumen segment 303, and a second portion 304 comprising a second lumen segment 305. The second portion is connected to the first portion by a hinge 306.

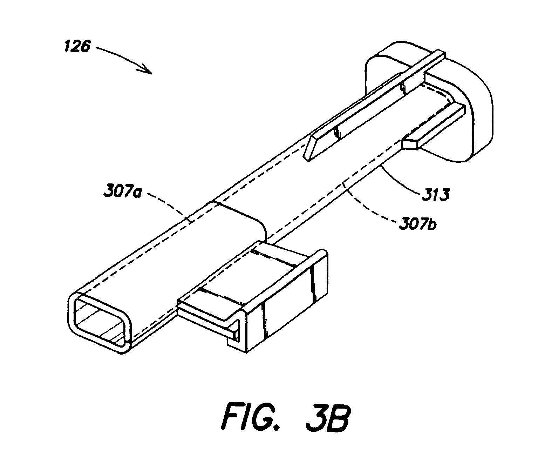

FIG. 3B is a perspective view of a closed cartridge of FIG. 3A following a rotation of first portion and/or second portion about hinge 306. A lumen portion 307a is formed between the first lumen portion 303 and the second lumen portion 305 upon rotation about the hinge 306. Proximal portion 313 includes a cartridge lumen portion 307b therethrough. The lumen portion 307b through portion 313 is aligned with lumen portion 307a such that a plunger can extend therethrough. The hinge may be constructed such that the angle between the first portion and the second portion can reach an angle of no more than 180 degrees when in an open state. Proximal portion 313 facilitates assembly and/or operation of an injector by providing a guide to a plunger tip during assembly and/or operation.

Upon rotating the first portion and second portion relative to one another, the IOL is manipulated into a folded state and the first lumen segment and the second lumen segment combine to form cartridge lumen portion 307a. Accordingly, after rotation, the IOL is folded and ready for insertion into a patient's eye upon connection of the cartridge with the remaining components of an injector. Additional information and detail of an IOL cartridge is described in U.S. patent application no. 2008/02 69 770 A1.

In one embodiment, prior to implementing the plunger device, a solution is added to the IOL injector, preferably through an orifice to wet the IOL as well as the interior surfaces of the cartridge 126 and inserter tip 125. The solution can be, for example, a saline solution, or a viscoelastic composition. Such hydration by the solution is effective to facilitate the lubricity enhancing characteristics of the hydrophilic coating component.

Having thus described the inventive concepts and a number of exemplary embodiments, it will be apparent to those skilled in the art that the invention may be implemented in various ways, and that modifications and improvements will readily occur to such persons. Thus, the embodiments are not intended to be limiting and presented by way of example only. The invention is limited only as required by the following claims and equivalents thereto.

### Examples

### Irradiation Apparatus

The AI28 and AI20 inserter t-cells, which are manufactured by Bausch & Lomb, Inc., were exposed to high energy UV-C light using an irradiation apparatus. The UV irradiation apparatus is a UVO-CLEANER® Model 342 manufactured by Jelight Company, Inc. of Irvine, CA. The UV irradiation apparatus **40** resembles an electric broiler with the UV lamp assembly **46** along the top side and a sliding drawer **42** equipped with a sample tray **44** (shown in open position). The t-cells are placed in the tray approximately six inches from the lamp assembly. The UV irradiation source is a low pressure mercury vapor grid with an output of 28,000 µW/cm at 254 nm (at 6 mm). The t-cells were exposed to this light in air or a flow of dioxygen for 12 minutes.

### X-ray Photoelectron Spectroscopy (XPS) Apparatus

A Physical Electronics Quantum 2000 Scanning ESCA Microprobe was used for the surface characterization of the t-cell before (control cells) and following UV irradiation. This instrument utilizes a monochromatic Al anode operated at 15kV and 0.25Watts/µm. All acquisitions were rastered over a 500 micron x 500 micron analysis area. Dual beam neutralization (ions and electrons) was used during all analysis. The base pressure of the instrument was 5 x 10⁻¹⁰ torr and during operation the pressure was less than or equal to 1 x 10⁻⁷ torr. This instrument made use of a hemispherical analyzer operated in FAT mode. A gauze lens was coupled to a hemispherical analyzer in order to increase signal throughput. Data was collected with an HP workstation running Windows XP and COMPASS v 6.0A. The instrument utilized MultiPak version 7.1 software for data analysis. Assuming the inelastic mean free path for a carbon 1s s photoelectron is 35 Å, the practical measure for sampling depth for this instrument at a sampling angle of 45° is approximately 75Å. The governing equation for sampling depth in XPS is: d = 3λ sin θ where d is the sampling depth, λ is the photoelectron inelastic mean free path and θ is the angie formed between the sample surface and the axis of the analyzer. Each specimen was analyzed utilizing a low-resolution survey spectra (0-1 100eV) to identify the elements present on the sample surface. Quantification of elemental compositions was completed by integration of the photoelectron peak areas. Analyzer transmission, photoelectron cross-sections and source angle correction were taken into consideration in order to give accurate atomic concentration values.

Ten (10) XPS measurements were taken along the length of an AI28 inserter tip. Two of the three inserter tips showed 100 at.% carbon along the entire length of the strip. The third tip appeared to be contaminated with SiO₂ as the carbon varied along the tip from about 83 at.% to about 92 at.%, oxygen from about 7 at.% to about 14 at.% and silicon from about 0.7 at.% to about 4 at.%. Following the described UV irradiation (12 minutes) all three tips showed near identical elemental composition at the surface, even the tip suspected of contamination. The UV irradiation was conducted in air and with a flow stream of oxygen at 34,5 kPa (5 PSI). Moreover, the elemental analysis for each tip was relatively consistent along the length of the tip.

For the UV irradiation in air, the carbon content at the surface from 92.2 at.% to 95.2 at.%, 91.2 at.% to 94.0 at.% and 89.3 at.% to 94.2 at.%, and the oxygen content at the surface from 4.9 at.% to 7.9 at.%, 6.0 at.% to 8.6 at.% and 5.8 at.% to 10.7 at.%, respectively, along the length of the tip.

For the UV irradiation in the O₂ flow, the carbon content at the surface from 91.9 at.% to 94.5 at.%, 90.1 at.% to 94.2 at.% and 91.1 at.% to 96.6 at.%, and the oxygen content from 5.5 at.% to 8.1 at.%, 5.8 at.% to 9.9 at.% and 3.4 at.% to 8.9 at.%, respectively, along the length of the tip.

### Hydrophilic Coating

The UV-exposed t-cells were manually dip coated into the listed hydrophilic coating solutions and held in the solution for about 5 seconds. The solution coated t-cells were dried in an oven at 85 °C for 1 hour. The effect of several factors on coating adhesion and lubricity was investigated including the coating composition, UV irradiation time and whether the t-cells were processed by ethylene oxide sterilization. The t-cells of Tables 1 and 3 did undergo an ethylene oxide sterilization process whereas the t-cells of Tables 2 and 4 did not. The coating compositions were provided in isopropanol (IPA) or ethanol (EtOH). Mid-power Akreos Adapt AO IOLs were used for testing lens deliveries.

**Table 1.**

| **Ex. No.** | **t-cell** | **# trials** | **coating composition** | **coating transfer rating** | **observations** |
|---|---|---|---|---|---|
| 1 Reference example | AI28 | 5 | 10% PVP | 1.5 | 3 smooth deliveries : 2 tears 2 lens rotations |
| 2 Reference example | AI20 | 5 | 10% PVP | 1.0 | 5 smooth deliveries : 1 microtear |
| 3 Reference example | AI28 | 4 | 7.5% PVP | 1.0 | 4 smooth deliveries : 1 microtear |
| 4 Reference example | AI20 | 5 | 7.5% PVP | 1.0 | 5 smooth deliveries |
| 5 | AI28 | 5 | 5% PVP 5% PVP-VA" | 1.0 | 5 smooth deliveries |
| 6 | AI20 | 5 | 5% PVP 5% PVP-VA^{a} | 1.0 | 5 smooth deliveries, 2 tears |
| 7 | AI28 | 5 | 7.5% PVP 2.5%PVP-VA³ | 1.0 | 5 smooth deliveries |
| 8 | AI20 | 5 | 7.5% PVP 2.5% PVP-VA* | 1.0 | 5 smooth deliveries |

| | | | | | |
|---|---|---|---|---|---|
| ^{a} The PVP-VA copolymer has a ratio of PVP:VA of 60:40. * Reference Example | | | | | |

**Table 2.**

| **Ex. No.** | **t-cell** | **#trials** | **coating composition** | **coating transfer rating** | **observations** |
|---|---|---|---|---|---|
| 9 Reference example | AI28 | 3 | 10% PVP | 0 | 3 smooth deliveries |
| 10 Reference example | AI20 | 3 | 10% PVP | 1.0 | 3 smooth deliveries; observed low delivery force |
| 11 | AI20 | 3 | 5% PVP 5% PVP-VA^{a} (IPA) | 1.0 | 3 smooth deliveries ; observed low delivery force |
| 12 | AI28 | 3 | 10% PVP-VA^{b} (EtOH) | 0 | 3 smooth deliveries |
| 13 | AI20 | 3 | 10% PVP-VA^{b} (EtOH) | 0 | 3 smooth deliveries |
| 14 | AI28 | 3 | 10% PVP-VA^{a} (IPA) | 0 | 3 smooth deliveries |
| 15 | AI20 | 3 | 10%PVP-VA^{a} (IPA) | 0 | 3 smooth deliveries |

| | | | | | |
|---|---|---|---|---|---|
| ^{a}The PVP-VA copolymer has a ratio of PVP:VA of 60:40. ^{b}The PVP-VA copolymer has a ratio of PVP:VA of 70:30. * Reference Example | | | | | |

**Table 3.**

| **Ex No.** | **t-cell** | **# trials** | **coating composition** | **coating transfer rating** | **observations** |
|---|---|---|---|---|---|
| 16 | AI28 | 8 | 7.5% PVP 2.5% PVP-VA^{a} | 1.0 | 5 smooth deliveries |
| 17 | AI20 | 5 | 7.5% PVP 2.5% PVP-VA^{a} | 0 | 5 smooth deliveries ; observed low delivery force |
| 18 Reference example | AI28 | 5 | 10% PVP | 1.0 | 5 smooth deliveries : observed low delivery force |
| 19 Reference example | AI20 | 5 | 10% PVP | 0 | 5 smooth deliveries |
| 20 | AI28 | 10 | 10% PVP-VA^{a} | 1.0 | 10 smooth deliveries |
| 21 | AI20 | 10 | 10% PVP-VA^{a} | 0 | 10 smooth deliveries: 1 micro tear |

| | | | | | |
|---|---|---|---|---|---|
| ^{a} The PVP-VA copolymer has a ratio of PVP:VA of 60:40. * Reference Example | | | | | |

### Example 22 to 24.

UV-C treated AI20 t-cells were coated with three different concentrations (Ex. 22, 10%; Ex. 23, 15%; and Ex. 24, 20%) of (60:40) PVP-vinyl acetate copolymer solution as described above. The injection force required to deliver Akreos MI-60 IOL was measured using an Instron test system. T-cells were not sterilized for this study. +20.0 D lenses and Amvisc Plus viscoelastic was used for all lens deliveries. The force data reported in Table 4 indicates that Example 24, i.e., a coating solution of 20% (60:40) PVP:VA required the least amount of force to deliver a lens (a relatively low average peak injection force of 317 gm-f along with a tighter standard deviation of 16). An average 581 gm-f peak force is required to deliver standard +20D MI60 lenses with a GMS/PP AI20 system (control). Accordingly, UV-C treated AI20 t-cells coated with the solution of Example 24 exhibits a reduction in delivery force of about 45%.

**Table 4.**

| Ex. No. | Force gm-f, N=5 | additive transfer | observations |
|---|---|---|---|
| 22 | 429 (72) | 0 | No lens damage/tear |
| 23 | 358(46) | 0 | No lens damage/tear |
| 24 | 317(16) | 0 | No lens damage/tear |
| non-coated (control) | 581 | 3 (high) | micro-tears at 6 o'-clock position on lens |

### Examples 25 to 27

An accelerated aging study was conducted to estimate the shelf-life of PVP:VA copolymer coated AI28 t-cells stored at 82°C over 6 days (this simulates at least 1 year of room temperature performance based on the accelerated shelf life prediction equation: tᵣₑₐₗ = t_{acc} X 2^{(Ta-22°C)/10}, where Ta is the accelerated temperature and t is time. The t-cells were exposed to UV-C radiation and manually dip coated with a PVP:VA (60:40) copolymer coating as described previously. The coated parts were dried in an oven at 85 °C for 1 hour before aging in an oven at 82 °C. Samples were tested at time 0, Day 3 (6 months RT) and Day 6 (12 months RT). Mid-power Akreos Adapt AO IOLs were used for testing lens deliveries. The 6-day test data reported in Table 5 shows that the coating was robust and performed exceptionally well under the aggressive test conditions.

**Table 5.**

| **Ex. No.** | **t-cell** | **# trials** | **coating composition** | **coating transfer rating** | **observations** |
|---|---|---|---|---|---|
| 25 | AI28 | 5 | 10% PVP-VA^{a} | 0 | 5 smooth deliveries, no optic damage |
| 26 | AI28 | 5 | 10% PVP-VA^{a} | 0 | 5 smooth deliveries, no optic damage |
| 27 | AI28 | 5 | 10% PVP-VA^{a} | 0 | 5 smooth deliveries, no optic damage |

| | | | | | |
|---|---|---|---|---|---|
| ^{a}The PVP-VA copolymer has a ratio of PVP:VA of 60:40. | | | | | |

### Examples 28 to 30

The procedure described in Examples 25 to 27 is repeated except that following the coating and 1-hour drying of the t-cells the t-cells were subjected to ethylene oxide sterilization. Accelerated aging study was conducted by placing the parts in an oven at 82 °C. Samples were tested at time 0, Day 3 (6 months RT) and Day 6 (12 months RT). Mid-power Akreos Adapt AO IOLs and Amvisc Plus viscoelastic were used for testing lens deliveries. The test data reported in Table 6 shows that the coating was robust and performed exceptionally well under the aggressive test conditions.

**Table 6.**

| **Ex. No.** | **t-cell** | **# trials** | **coating composition** | **coating transfer rating** | **observations** |
|---|---|---|---|---|---|
| 28 | AI28 | 5 | 10% PVP-VA^{a} | 1 | 5 smooth deliveries, no optic damage |
| 29 | AI28 | 5 | 10% PVP-VA^{a} | 1 | 4 smooth deliveries. 1 scratch at 3 o'clock on anterior side of lens |
| 30 | AI28 | 5 | 10% PVP-VA^{a} | 1 | 5 smooth deliveries, no optic damage |

| | | | | | |
|---|---|---|---|---|---|
| ^{a} The PVP-VA copolymer has a ratio of PVP:VA of 60:40. | | | | | |

### Examples 31 to 33

The procedure described in Examples 28 to 30 is repeated except that a 20% PVP-VA copolymer solution was used to coat the t-cells. The test data reported in Table 7 shows that the coating was robust and performed exceptionally well under the aggressive test conditions.

**Table 7.**

| **Ex. No.** | **t-cell** | **# trials** | **coating composition** | **coating transfer rating** | **observations** |
|---|---|---|---|---|---|
| 31 | AI28 | 5 | 20% PVP-VA^{a} | 1 | 5 smooth deliveries, no optic damage |
| 32 | AI28 | 5 | 20% PVP-VA^{a} | 1 | 5 smooth deliveries, no optic damage |
| 33 | AI28 | 5 | 20% PVP-VA^{a} | 1 | 5 smooth deliveries, no optic damage |

| | | | | | |
|---|---|---|---|---|---|
| ^{a} The PVP-VA copolymer has a ratio of PVP:VA of 60:40. | | | | | |

## Claims

1. An intraocular lens injector prepared by a process comprising:
irradiating at least a portion of a polymeric, intraocular lens injector with UV light in an environment comprising oxygen to provide a positive percent change in the atomic oxygen content of the polymer material at the surface as determined by X-ray photoelectron spectroscopy;
contacting at least a portion of the irradiated portion with a solution comprising a hydrophilic copolymer selected from poly(PVP-VA), poly(PVP-AA) or poly(PVP-DMA) to provide a solution coated portion; and
heating the solution coated portion to provide the lens injector with a shelf-stable, lubricious coating to facilitate delivery of an intraocular lens from the injector with minimal damage to the lens and with little or no transfer of the lubricious coating to a surface of the lens.

2. The intraocular lens injector of claim 1 further comprising:
contacting at least a portion of the irradiated portion with a solution comprising a crosslinkable polymer and initiating the crosslinking with light or heat prior to contacting at least a portion of the irradiated portion with the solution comprising the hydrophilic copolymer.

3. The intraocular lens injector of claims 1 or 2 wherein the UV light is UV-C light.

4. The intraocular lens injector of any one of claims 1 to 3 wherein the hydrophilic copolymer is poly(PVP-VA) with a PVP:VA ratio of 30:70 to 80:20.

5. The intraocular lens injector of any one of claims 1 to 4 wherein the portion of a polymeric, intraocular lens injector that is irradiated with UV light comprises polypropylene, and the atomic oxygen content of the polymer material at the surface of said irradiated portion prior to contacting said portion with a solution comprising the hydrophilic copolymer, is from 5 at.% to 20 at.% as determined by X-ray photoelectron spectroscopy.

6. The intraocular lens injector of any one of claims 1 to 5 wherein the ; shelf-stable, lubricious coating exhibits greater shelf-stability than a similar lubricious coating applied without irradiation with the UV light.

7. An intraocular lens injector of any one of claims 1 to 6 wherein the heating of the solution coated injector tip is conducted at a temperature from 35 °C to 110°C.

## Patentansprüche

1. Ein Intraokularlinseninjektor, hergestellt durch ein Verfahren, umfassend:
Bestrahlen von mindestens einem Teil eines polymeren Intraokularlinseninjektors mit UV-Licht in einer Umgebung, welche Sauerstoff umfasst, um eine positive prozentuale Veränderung im atomaren Sauerstoffgehalt des Polymermaterials an der Oberfläche, bestimmt durch Röntgen-Photoelektronenspektroskopie, bereitzustellen;
Kontaktieren von mindestens einem Teil des bestrahlten Teils mit einer Lösung, welche ein hydrophiles Copolymer, ausgewählt aus Poly(PVP-VA), Poly(PVP-AA) oder Poly(PVP-DMA), umfasst, um einen mit Lösung beschichteten Teil bereitzustellen; und
Erwärmen des mit Lösung beschichteten Teils, um den Linseninjektor mit einer lagerstabilen, gleitfähigen Beschichtung zu versehen, welche die Abgabe einer Intraokularlinse von dem Injektor unter minimaler Beschädigung der Linse und mit wenig oder keiner Übertragung der gleitfähigen Beschichtung auf eine Oberfläche der Linse ermöglicht.

2. Der Intraokularlinseninjektor gemäß Anspruch 1, weiterhin umfassend:
Kontaktieren von mindestens einem Teil des bestrahlten Teils mit einer Lösung, welche ein vernetzbares Polymer umfasst, und Starten des Vernetzens durch Licht oder Wärme vor dem Kontaktieren von mindestens einem Teil des bestrahlten Teils mit der Lösung, welche das hydrophile Copolymer umfasst.

3. Der Intraokularlinseninjektor gemäß Anspruch 1 oder 2, wobei das UV-Licht UV-C-Licht ist.

4. Der Intraokularlinseninjektor gemäß einem der Ansprüche 1 bis 3, wobei das hydrophile Copolymer Poly(PVP-VA) ist, welches ein PVP:VA Verhältnis von 30:70 bis 80:20 aufweist.

5. Der Intraokularlinseninjektor gemäß einem der Ansprüche 1 bis 4, wobei der Teil des polymeren Intraokularlinseninjektors, welcher mit UV-Licht bestrahlt wird, Polypropylen umfasst, und der atomare Sauerstoffgehalt des Polymermaterials an der Oberfläche des bestrahlten Teils vor dem Kontaktieren des Teils mit einer Lösung, welche das hydrophile Copolymer umfasst, von 5 At.% bis 20 At.%, bestimmt durch Röntgen-Photoelektronenspektroskopie, beträgt.

6. Der Intraokularlinseninjektor gemäß einem der Ansprüche 1 bis 5, wobei die lagerstabile, gleitfähige Beschichtung eine höhere Lagerstabilität aufweist als eine ähnliche gleitfähige Beschichtung, welche ohne Bestrahlung mit dem UV-Licht aufgetragen ist.

7. Ein Intraokularlinseninjektor gemäß einem der Ansprüche 1 bis 6, wobei das Erwärmen der mit Lösung beschichteten Injektorspitze bei einer Temperatur von 35° bis 110°C durchgeführt wird.

## Revendications

1. Injecteur de lentille intraoculaire préparé au moyen d'un procédé comprenant :
l'irradiation d'au moins une partie d'un injecteur de lentille intraoculaire polymère à la lumière UV dans un environnement comprenant de l'oxygène pour fournir un changement en pourcentage positif de la teneur atomique en oxygène du matériau polymère à la surface comme déterminé par la spectroscopie des photoélectrons X ;
la mise en contact d'au moins une partie de la partie irradiée avec une solution comprenant un copolymère hydrophile choisi parmi le poly(PVP-VA), le poly(PVP-AA) ou le poly(PVP-DMA) pour fournir une partie enduite de solution ; et
le chauffage de la partie enduite de solution pour doter l'injecteur de lentille d'un revêtement lubrifié longue conservation pour faciliter la fourniture d'une lentille intraoculaire à partir de l'injecteur avec un endommagement minimal de la lentille et avec un transfert infime, voire nul, du revêtement lubrifié vers une surface de la lentille.

2. Injecteur de lentille intraoculaire selon la revendication 1, comprenant en outre :
la mise en contact d'au moins une partie de la partie irradiée avec une solution comprenant un polymère réticulable et l'amorce de la réticulation à la lumière ou à la chaleur avant la mise en contact d'au moins une partie de la partie irradiée avec la solution comprenant le copolymère hydrophile.

3. Injecteur de lentille intraoculaire selon les revendications 1 ou 2, dans lequel la lumière UV est une lumière UV-C.

4. Injecteur de lentille intraoculaire selon l'une quelconque des revendications 1 à 3, dans lequel le copolymère hydrophile est un poly(PVP-VA) avec un rapport PVP:VA de 30:70 à 80:20.

5. Injecteur de lentille intraoculaire selon l'une quelconque des revendications 1 à 4, dans lequel la partie d'un injecteur de lentille intraoculaire polymère qui est irradiée à la lumière UV comprend du polypropylène, et la teneur atomique en oxygène du matériau polymère à la surface de ladite partie irradiée, avant la mise en contact de ladite partie avec une solution comprenant le copolymère hydrophile, est de 5 % atomique à 20 % atomique, comme déterminé par la spectroscopie de photoélectrons X.

6. Injecteur de lentille intraoculaire selon l'une quelconque des revendications 1 à 5, dans lequel le revêtement lubrifié longue conservation présente une stabilité de conservation supérieure à un revêtement.lubrifié similaire appliqué sans irradiation à la lumière UV.

7. Injecteur de lentille intraoculaire selon l'une quelconque des revendications 1 à 6, dans lequel le chauffage de la pointe d'injecteur enduite de solution est opéré à une température de 35°C à 110°C.
